# FASCICULE DE BREVET EUROPEEN

(11) **EP 0 577 820 B1**
(45) Date de publication et mention de la délivrance du brevet: **20.03.1996**
(21) Numéro de dépôt: 93904141.4
(22) Date de dépôt: 29.01.1993
(51) Int. Cl.: A61K 7/00, A61K 7/021

(54) **COMPOSITION COSMETIQUE POUR LE MAQUILLAGE CONTENANT UN PIGMENT TRANSPARENT D'OXYDE DE TITANE ET D'OXYDE DE SILICIUM**
EIN DURCHSICHTIGES PIGMENT AUS TITANOXYD UND SILIZIUMOXYD ENTHALTENDE SCHMINKE
COSMETIC MAKE-UP COMPOSITION CONTAINING A TRANSPARENT TITANIUM OXIDE AND SILICON OXIDE PIGMENT

(30) Priorité: 31.01.1992 FR 9201086
(43) Date de publication de la demande: 12.01.1994
(73) Titulaire: L'OREAL, F-75008 Paris (FR)
(72) Inventeur: DEFOSSEZ, Béatrice, F-75020 Paris (FR); ROSSIGNOL, Sylvie, F-75014 Paris (FR)
(74) Mandataire: Stalla-Bourdillon, Bernard
(86) Numéro de dépôt international: FR9300099
(87) Numéro de publication internationale: WO9314739

(56) Documents cités:
- EP-A- 0 268 938
- EP-A- 0 377 326
- GB-A- 2 226 018
- S.T.N., Serveur de Bases de Données, Karlsruhe, DE, Fichier Chemical Abstracts, volume: 111, no. 180453 & JP-A-01 056 607, publié le 03.03.89 Shiseido

## Description

La présente invention a pour objet une composition cosmétique pour le maquillage, celle-ci contenant un pigment transparent d' oxyde de titane et d'oxyde de silicium.

Les produits de maquillage tels que les poudres libres ou compactées, les fonds de teint, les fards à joues, les fards à paupières, les rouges à lèvres ainsi que les vernis à ongles contiennent en des proportions variables, des pigments destinés à conférer à ces produits, une certaine couleur ainsi qu'éventuellement des charges minérales destinées notamment à apporter un bon pouvoir couvrant.

En vue d'obtenir un effet couvrant, on peut utiliser du dioxyde de titane en tant que pigment blanc opacifiant. Toutefois, son utilisation rend difficile l'étalement des compositions sur la peau et entraîne son blanchiment. Si l'on recherche en outre l'obtention d'un effet nacrant, il faut rajouter un pigment nacrant tel que du mica recouvert d'oxyde de titane (mica-titane) ou de l'oxychlorure de bismuth. L'emploi de mica-titane peut néanmoins présenter des désavantages liés au mauvais étalement et au manque de douceur.

Par ailleurs, lorsque l'on souhaite renforcer l'effet nacrant, il est possible d'augmenter le taux de nacre mais l'on rencontre alors des problèmes de cohésion qui nécessitent l'augmentation du taux de liant de manière telle que cela entraîne des inconvénients lors de l'application. Enfin, il convient de noter que les nacres sont des particules particulièrement fragiles qui, lorsqu'elles sont broyées, perdent leur effet nacrant.

Si l'on désire conférer un effet coloré prononcé, il est nécessaire d'utiliser des pigments organiques ou minéraux colorés, mais l'augmentation du taux des pigments provoque d'une part, une saturation de couleur et, d'autre part, des problèmes d'homogénéité des pigments dans la formule.

Si l'on souhaite renforcer l'effet coloré des pigments, on peut incorporer comme ceci est décrit dans la demande de brevet JP 61-295234, de l'oxyde de fer, par exemple, dans la couche d'enrobage du dioxyde de titane lamellaire.

Après diverses études en vue de remédier aux inconvénients mentionnés ci-dessus, on a constaté de façon inattendue qu'en utilisant à titre d'agent nacrant dans une composition cosmétique de maquillage, un pigment transparent constitué d'au moins 60 % en poids d'oxyde de titane et au plus de 10 % d'oxyde de silicium, il était possible d'obtenir d'excellentes propriétés cosmétiques, ces pigments transparents conférant à la fois un nacrage satisfaisant et un niveau de couvrance approprié, une bonne aptitude à l'étalement et une douceur que l'on n'observe pas avec les pigments nacrés classiques. On obtient également un film plus homogène dans lequel les pigments transparents sont bien répartis.

Ces pigments transparents, du fait de leur effet brillant, nacré et irisé coloré, et de par leurs propriétés cosmétiques, sont particulièrement favorables à un usage cosmétique sans que l'on rencontre les inconvénients des pigments de l'état de la technique. De plus, ces pigments transparents sont moins fragiles que les nacres, ce qui permet de les broyer sans perdre leur effet nacrant.

La présente invention a donc pour objet une composition cosmétique pour le maquillage contenant dans un support cosmétique approprié, un pigment transparent constitué d'au moins 60 % en poids d'oxyde de titane sous forme lamellaire et d'au plus 10 % en poids d'oxyde de silicium, la dimension moyenne des pigments étant comprise entre 1 et 300 µm et l'épaisseur entre 0,001 à 0,3 µm.

De préférence, les pigments transparents sont constitués d'au moins 90 % en poids d'oxyde de titane sous forme lamellaire, et d'au plus 6 % en poids d'oxyde de silicium.

Selon une forme de réalisation préférée de l'invention, la dimension moyenne des pigments transparents est comprise entre 5 et 50 µm, et l'épaisseur entre 0,01 et 0,2 µm et plus particulièrement entre 0,1 et 0,2 µm.

Les pigments transparents de la composition cosmétique selon l'invention sont décrits dans la demande de brevet européen EP 377 326 à laquelle il est fait ici référence. Ces pigments transparents sont, selon cette demande, obtenus par dissolution d'une majeure partie de l'oxyde de silicium de particules d'oxyde de silicium revêtues d'oxyde de titane transparent, en choisissant des particules d'oxyde de silicium pour lesquelles une majeure partie de l'oxyde de silicium est soluble dans une base telle que la soude ou la potasse.

Comme pigments transparents d'oxyde de titane et d'oxyde de silicium, on peut utiliser ceux vendus par la Société KEMIRA sous les dénominations de FLONAC R "TS40C" "TS50C" "TS60C" et "TS70C".

Le produit "TS40C" est constitué de 94 % d'oxyde de titane et de 4 % d'oxyde de silicium et se présente sous forme d'une poudre cristalline présentant des reflets rouges.

Le produit "TS50C" est constitué de 94 % d oxyde de titane et de 4 % d'oxyde de silicium et se présente sous forme d'une poudre cristalline présentant des reflets lilas.

Le produit "TS60C" est constitué de 94 % d'oxyde de titane et de 4 % d'oxyde de silicium et se présente sous forme d'une poudre cristalline présentant des reflets bleus.

Le produit "TS70C" est constitué de 94 % d oxyde de titane et de 4 % d'oxyde de silicium et se présente sous forme d'une poudre cristalline présentant des reflets verts.

Dans les produits de maquillage selon l'invention, le pigment transparent tel que défini ci-dessus est généralement présent à une concentration comprise entre 0,5 et 30 % et de préférence entre 0,5 et 10 % en poids par rapport au poids total de la composition.

Les compositions cosmétiques selon l'invention peuvent être utilisées comme produits de maquillage de la peau, des yeux ou des cheveux.

Lorsque les compositions cosmétiques sont destinées au maquillage de la peau, celles-ci sont essentiellement des fards à paupières, des eye-liners, des mascaras, des poudres, des fonds de teint, des fards à joues, des "blush", des crèmes teintées, des rouges à lèvres, des sticks à lèvres ou des sticks anti-cernes.

Les pigments transparents trouvent également une application dans les vernis à ongles.

De façon préférentielle, les compositions cosmétiques selon l'invention sont plus particulièrement destinées au maquillage des yeux, c'est-à-dire sous la forme de fards à paupières, d'eye-liners ou de mascaras.

Les compositions cosmétiques de maquillage selon l'invention peuvent en outre contenir d'autres pigments et/ou des charges additionnels usuels en cosmétique.

Parmi les pigments minéraux, on peut citer les oxydes métalliques obtenus par voie synthétique ou d'origine naturelle tels que l'oxyde de fer, de titane, de zinc, de chrome, etc, et à titre d'exemples :
- le dioxyde de titane (rutile ou anatase) éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome hydraté (CI 77289) ;
- le bleu ferrique (CI 77510).
et tous les pigments minéraux ayant subi un traitement de surface minéral ou organique.

Parmi les pigments organiques, on peut citer, en particulier, les suivants :
- D & C red n° 19 (CI 45170) ;
- D & C red n° 9 (CI 15585) ;
- D & C red n° 21 (CI 45380) ;
- D & C orange n° 4 (CI 15510) ;
- D & C orange n° 5 (CI 45370) ;
- D & C red n° 27 (CI 45410) ;
- D & C red n° 13 (CI 15630) ;
- D & C red n° 7 (CI 15850-1) ;
- D & C red n° 6 (CI 15850-2) ;
- D & C yellow n° 5 (CI 19140) ;
- D & C red n° 36 (CI 12085) ;
- D & C orange n° 10 (CI 45425) ;
- D & C yellow n° 6 (CI 15985) ;
- D & C red n° 30 (CI 73360) ;
- D & C red n° 3 (CI 45430) ;
- le noir de carbone (CI 77266) ; et
- les laques à base de carmin de cochenille (CI 75470).

Selon l'invention les pigments additionnels peuvent représenter de 0,01 à 30 % en poids de la composition, de préférence de 0,2 à 15 %.

Les charges sont choisies notamment parmi :
- la silice,
- la séricite,
- le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoelithe, lépidolithe, biotite) ou d'origine synthétique. Les micas sont généralement transparents et permettent de conférer à la peau un aspect satiné ;
- l'amidon, en particulier I'amidon de riz ;
- le kaolin, qui est un silicate d'aluminium hydraté, qui se présente sous la forme de particules de forme isotrope ayant des dimensions généralement inférieure à 30 µm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 µm dans le cas de l'oxyde de titane) : ces oxydes ont un toucher onctueux, un bon pouvoir couvrant et une opacité importante ;
- le nitrure de bore ;
- le carbonate de calcium précipité qui, sous forme de particules de dimensions inférieures à 10 µm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, etc. Ces savons, présents généralement sous la forme de particules ayant des dimensions inférieures de 10 µm ont un toucher onctueux et facilitent l'adhérence de la poudre sur la peau ;
- Les poudres de polymères synthétiques, tels que le polyéthylène, les polyesters (par exemple isophtalate ou téréphtalate de polyéthylène), les polyamides, sous la forme de particules ayant des dimensions inférieures à 50 µm, qui possèdent des propriétés absorbantes, et permettent de conférer à la peau un aspect velouté, les poudres de méthacrylate ;
- des microsphères creuses, telles que par exemple les microsphères creuses d'Expancel vendues par la Société Kemanord Plast AB et décrites dans le brevet français 86 09289 (2.600.532).

Selon l'invention les charges additionnelles peuvent représenter de 0,01 à 90 % en poids de la composition.

Les compositions selon la présente invention peuvent notamment se présenter sous forme d'émulsion huile-dans-eau ou eau-dans-huile, ou sous forme d'une suspension en milieu solvant, ou encore sous forme de poudre libre, de poudre compactée ou de pâte anhydre. Les modes opératoires pour la préparation de ces différents types de compositions sont bien connus de l'homme de l'art.

Lorsqu'elles sont utilisées sous forme d'émulsion, les compositions peuvent contenir des agents tensio-actifs bien connus dans l'état de la technique. Ces tensio-actifs peuvent constituer de 0,01 à 30 % en poids de la composition.

Une réalisation particulièrement préférée consiste à préparer des émulsions anioniques ou non-ioniques en utilisant des agents tensio-actifs anioniques ou non-ioniques, tels que ceux énumérés dans la demande de brevet français n° 2.629.713, dans des proportions de préférence comprises entre 2 et 30 % en poids par rapport au poids total de la composition.

Dans ces émulsions, la phase huileuse peut représenter de 0,1 à 50 % en poids de l'émulsion. Elle peut être constituée d'huiles et/ou de cires bien connues dans l'état de la technique. Les cires et les huiles peuvent être d'origines végétale, animale, minérale ou synthétique.

La phase huileuse peut par ailleurs contenir des colorants, des filtres solaires, des antioxydants, des conservateurs, et des principes actifs lipophiles.

Lorsque l'émulsion contient un filtre solaire, notamment dans des proportions allant de 0,5 à 15 % en poids par rapport au poids total de la composition, les pigments transparents définis ci-dessus apportent un aspect esthétique à la composition filtrante.

Les compositions conformes à la présente invention peuvent contenir, en plus des composants mentionnés ci-dessus, des ingrédients classiquement utilisés, notamment dans les compositions de maquillage, et choisis parmi les adoucissants, les conservateurs, les séquestrants, les parfums, les épaississants, les agents de cohésion, les polymères ainsi que les agents alcalinisants ou acidifiants habituellement utilisés dans le domaine cosmétique, et des hydratants.

Les épaississants utilisables peuvent être naturels ou synthétiques. Parmi les épaississants naturels, on peut citer les gommes de diverses sortes telles que les gommes arabique, de guar ou de caroube. Parmi les épaississants de synthèse, on peut citer les dérivés cellulosiques comme l'hydroxyéthylcellulose, la carboxyméthylcellulose, les dérivés de l'amidon, les dérivés d'éthers de cellulose possédant des groupes ammonium quaternaires, les polysaccharides cationiques, les sels de polymères acryliques ou méthacryliques.

On peut également obtenir un épaississant des compositions par mélange de polyéthylèneglycol et de stéarate et/ou de distéarate de polyéthylèneglycol ou par un mélange d'esters phophoriques et d'amides gras.

Ces agents épaississants sont notamment utilisés comme agent de suspension des pigments et facilitent la répartition homogène du pigment dans la composition. Ils conviennent donc pour des compositions se présentant sous forme de suspension pour la coloration temporaire des fibres kératiniques, en particulier les cheveux, éliminables au premier shampooing.

Selon l'invention les compositions anhydres qui peuvent se présenter sous forme de poudre libre ou compactée, de fard solide, pâteux ou liquide peuvent contenir un liant qui peut représenter de préférence de 0,01 à 95 % en poids.

Parmi les agents liants, on peut citer notamment les huiles animales, végétales ou synthétiques,les mélanges d'huile(s) et de cire(s) et en particulier l'huile de vison, l'huile de tortue, l'huile de soja, l'huile de pépins de raisin, l'huile de sésame, l'huile de maïs, l'huile de colza, l'huile de tournesol,l'huile de coton, l'huile d'avocat, l'huile d'olive, l'huile de ricin, l'huile de jojoba, l'huile d'arachide, etc... ; des huiles d'hydrocarbures, telles que des huiles de paraffine, le squalane, la vaseline, etc... ; des esters tels que le myristate d'isopropyle, le palmitate d'isopropyle, le stéarate de butyle, le laurate d'hexyle, l'isononanate d'isononyle, le palmitate de 2-éthyl hexyle, le laurate de 2-hexyl-décyle, le palmitate de 2-octyl-décyle, le myristate de 2-octyl-dodécyle, le succinate de 2-di-éthyl-hexyle, le malate de diisostéaryle, le lactate de 2-octyl-dodécyle, le triisostéarate de glycérine, le triisostéarate de diglycérine, etc... ; des huiles de silicone comme les poly-méthylsiloxanes, les poly-méthylphénylsiloxanes, des polysiloxanes modifiés par des acides gras, des polysiloxanes modifiés par des alcools gras, des polysiloxanes modifiés par des polyoxyalkylènes, des silicones fluorées, etc... ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, etc... ; des alcools gras supérieurs tels que le cétanol, l'alcool stéarylique, l'alcool oléique, etc... ; les cires peuvent être choisies notamment parmi la cire de carnauba, la cire de candelilla, la cire d'abeille, la cire de baleine, des lanolines, des cires microcristallines, etc...

Le liant peut contenir en outre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 0,5.10²Pa).

Parmi les huiles volatiles pouvant être présentes comme agents d'étalement dans la composition de l'invention, on citera par exemple les huiles de silicone telles que l'hexaméthyldisiloxane, le cyclopentadiméthylsiloxane, le cyclotétraméthylsiloxane, des huiles fluorées comme celle commercialisée sous la dénomination GALDEN (MONTEFLUOS) ou des huiles isoparaffiniques telles que celles commercialisées sous la dénomination ISOPAR (E, G, L ou H).

Comme mentionné ci-dessus, les compositions selon l'invention peuvent également se présenter sous forme d'un vernis à ongles contenant le pigment transparent en une proportion de 0,01 à 10 % en poids.

Le système solvant du vernis représente généralement de 55 à 90 % en poids du poids total du vernis.

Ce système solvant est constitué par un mélange de divers solvants organiques volatils tels que l'acétone, l'acétate d'éthyle, l'acétate de butyle, l'acétate de méthoxy-2 éthyle, la méthyléthylcétone, la méthylisobutylcétone, l'acétate de méthyle, l'acétate d'amyle et l'acétate d'isopropyle.

Le système solvant peut également comprendre un diluant tel que l'hexane ou l'octane ou encore un hydrocarbure aromatique tel que le toluène ou le xylène en une proportion de 10 à 35 % en poids par rapport au poids total du vernis.

La matière filmogène du vernis est généralement présente à une concentration comprise entre 5 et 20 % en poids par rapport au poids total du vernis.

Parmi les matières filmogènes on peut notamment citer les nitrocelluloses du type "RS" ou "SS" et en particulier la nitrocellulose type 1/4"RS" la nitrocellulose type 1/2"RS" la nitrocellulose type 1/2"SS" et la nitrocellulose type 3/4"RS".

Les vernis contiennent également un agent plastifiant généralement présent à une concentration comprise entre 2 et 10 % en poids par rapport au poids total du vernis. Parmi ceux-ci on peut notamment mentionner le phosphate de tricrésyle, le benzoate de benzyle, le citrate de triéthyle, le citrate de tributyle, l'acétylcitrate de triéthyle, l'acétylcitrate de triétyl-2 hexyle, le phtalate de diamyle, le camphre.

Les vernis selon l'invention contiennent également une résine généralement présente à une concentration comprise entre 0,5 et 15 % en poids par rapport au poids total du vernis.

Parmi les nombreuses résines utilisables on peut notamment citer les résines du type aryl-sulfonamide formaldéhyde ou aryl-sulfonamide époxy notamment les résines connues sous les dénominations commerciales "SANTOLITE MHP" et "SANTOLITE MS 80 %".

Les vernis à ongles selon l'invention peuvent également contenir des adjuvants couramment utilisés dans les vernis à ongles tels que par exemple des filtres U.V.

On va maintenant donner à titre d'illustration et sans aucun caractère limitatif, plusieurs exemples de compositions cosmétiques de maquillage selon l'invention.

### EXEMPLE 1 : Fard à paupières

| | |
|---|---|
| - Mica | 10,0 g |
| - Poudre de nylon vendue sous la dénomination de "ORGASOL" par la Société Atochem | 15,0 g |
| - Stéarate de zinc | 3,0 g |
| - Oxyde de fer rouge | 0,5 g |
| - Oxyde de fer jaune | 2,0 g |
| - Pigment d'oxyde de titane et de silice vendu sous la dénomination de FLONAC R "TS40" par la Société KEMIRA | 5,0 g |
| - Huile de vaseline | 3,0 g |
| - Vaseline | 0,6 g |
| - Lanoline | 0,6 g |
| - Huile de ricin | 1,8 g |
| - Talc q.s.p. | 100 g |

Pour comparaison, on reproduit la même formule en remplaçant le pigment FLONAC R "TS40" par du mica-titane.

La composition selon l'invention est supérieure à celle obtenue avec du mica-titane du point de vue facilité d'étalement, homogénéité, douceur et couvrance.

### EXEMPLE 2 : Fard à paupières

| | |
|---|---|
| - Mica | 20,0 g |
| - Stéarate de zinc | 3,0 g |
| - Oxyde de fer noir | 0,5 g |
| - Oxyde de fer jaune | 2,0 g |
| - Pigment d'oxyde de titane et de silice vendu sous la dénomination de FLONAC R "TS70C" par la Société KEMIRA | 5,0 g |
| - Huile de vaseline | 5,4 g |
| - Alcool oléique | 1,0 g |
| - Vaseline | 1,0 g |
| - Myristate d'isopropyle | 0,8 g |
| - Parahydroxybenzoate de propyle | 0,2 g |
| - Talc q.s.p. | 100 g |

Pour comparaison, on reproduit la même formule en remplaçant le pigment FLONAC R "TS70C" par de l'oxychlorure de bismuth.

La composition selon l'invention présente une cohésion supérieure et au niveau maquillage, on a un effet coloré irisé qu'on n'obtient pas en présence de l'oxychlorure de bismuth.

### EXEMPLE 3 : Fard à joues

| | |
|---|---|
| - Microsphères de poly-β-alanine réticulée imprégnées de glycérol à 300 % | 4,0 g |
| - Petrolatum vendu sous la dénomination "SERAFINE 56/58" par la Société SERESINE | 10,0 g |
| - Cire de carnauba | 5,0 g |
| - Cire de polyéthylène | 5,0 g |
| - Pigment d'oxyde de titane et de silice vendu sous la dénomination FLONAC R "TS40C" par la Société KEMIRA | 10,0 g |
| - Dioxyde de titane | 5,0 g |
| - D and C Red n°7 vendu sous la dénomination "WOO5" par la Société WACKHERR | 0,2 g |
| - Oxydes de fer noir qs | |
| - Oxyde de fer jaune qs | |
| - Oxyde de fer rouge qs | |
| - Parahydroxybenzoate de propyle | 0,1 g |
| - Butylhydroxytoluène | 0,05 g |
| - Huile de paraffine vendue sous la dénomination de "MARCOL 82" par la Société ESSO q.s.p. | 100 g |

### EXEMPLE 4 : Vernis à ongles coloré

| | |
|---|---|
| - Nitrocellulose | 10,62 g |
| - Résine toluène sulfonamide formaldéhyde vendue sous la dénomination "KETJENFLEX MS80" par la Société AKZO | 9,54 g |
| - Acétylcitrate de tributyle vendu sous la dénomination de "CITROFLEX A4" par la Société PFIZER | 6,32 g |
| - Toluène | 30,28 g |
| - Acétate de butyle | 21,13 g |
| - Acétate d'éthyle | 9,15 g |
| - Alcool isopropylique | 7,60 g |
| - Stéaralkonium hectorite vendu sous la dénomination de "BENTONE 27" par la Société NL CHEMICALS | 1,35 g |
| - Pigments | 1,0 g |
| - Pigment d'oxyde de titane et de silice vendu sous la dénomination FLONAC R de "TS40C" par la Société KEMIRA | 1,0 g |
| - Acide citrique | 0,06 g |

### EXEMPLE 5 : Fond de teint

| | |
|---|---|
| - Stéarate de glycérol | 2,2 g |
| - Triglycérides d'acides caprique/caprylique vendus sous la dénomination de "MIGLYOL 812" par la Société DYNAMIT NOBEL | 15,0 g |
| - Oxydes de fer jaune | 0,75 g |
| - Oxydes de fer brun | 0,47 g |
| - Oxyde de fer noir | 0,23 g |
| - Dioxyde de titane | 4,55 g |
| - Parahydroxybenzoate de méthyle | 0,1 g |
| - Parahydroxybenzoate de propyle | 0,1 g |
| - Imidazolidinyl urée | 0,3 g |
| - 2-hydroxy 4-méthoxybenzophénone | 0,5 g |
| - N,N-diméthylparaaminobenzoate d'octyle | 0,5 g |
| - Pigment d'oxyde de titane et de silice vendu sous la dénomination de FLONAC R "TS40C" par la Société KEMIRA | 3,0 g |
| - Silicate d'aluminium et de magnésium vendu sous la dénomination "VEEGUM" par la Société VENDERBILT | 1,0 g |
| - Triéthanolamine | 1,0 g |
| - Gomme de cellulose | 0,16 g |
| - Sel d'aluminium du produit de réaction de l'anhydride octénylsuccinique avec l'amidon vendu sous la dénomination de "DRY FLO" par la Société NATIONAL STARCH | 5,0 g |
| - Cyclométhicone vendue sous la dénomination de "VOLATIL SILICONE 7158" par la Société UNION CARBIDE | 10,0 g |
| - Eau | 47,34 g |
| - Propylène glycol | 2,0 g |
| - Glycérine | 3,0 g |
| - Sel de sodium de lauroyl sarcosine vendu sous la dénomination "ORAMIX L30" par la Société SEPPIC | 0,6 g |
| - Acide stéarique | 2,2 g |

### EXEMPLE 6 : Fond de teint

| | |
|---|---|
| - Stéarate de glycérol | 2,2 g |
| - Triglycérides d'acides caprique/caprylique vendus sous la dénomination "MIGLYOL 812" par la Société DYNAMIT NOBEL | 15,0 g |
| - Oxydes de fer jaune | 0,75 g |
| - Oxydes de fer brun | 0,47 g |
| - Oxyde de fer noir | 0,23 g |
| - Dioxyde de titane | 4,55 g |
| - Parahydroxybenzoate de méthyle | 0,1 g |
| - Parahydroxybenzoate de propyle | 0,1 g |
| - Imidazolidinyl urée | 0,3 g |
| - 2-hydroxy 4-méthoxybenzophénone | 0,5 g |
| - N,N-diméthylparaaminobenzoate d'octyle | 0,5 g |
| - Pigment d'oxyde de titane et de silice vendu sous la dénomination de FLONAC R "TS70C" par la Société KEMIRA | 3,0 g |
| - Silicate d'aluminium et de magnésium vendu sous la dénomination de "VEEGUM" par la Société VENDERBILT | 1,0 g |
| - Triéthanolamine | 1,0 g |
| - Gomme de cellulose | 0,16 g |
| - Sel d'aluminium du produit de réaction de l'anhydride octénylsuccinique avec l'amidon vendu sous la dénomination de "DRY FLO" par la Société NATIONAL STARCH | 5,0 g |
| - Cyclométhicone vendue sous la dénomination de "VOLATIL SILICONE 7158" par la Société UNION CARBIDE | 10,0 g |
| - Eau | 47,34 g |
| - Propylène glycol | 2,0 g |
| - Glycérine | 3,0 g |
| - Sel de sodium de lauroyl sarcosine vendu sous la dénomination de "ORAMIX L30" par la Société SEPPIC | 0,6 g |
| - Acide stéarique | 2,2 g |

### EXEMPLE 7 : Fard à paupières

| | |
|---|---|
| - Oxyde de fer | 10,7 g |
| - D & C Red n° 30 | 0,02 g |
| - Mica | 20 g |
| - Oxychlorure de bismuth | 14 g |
| - Poudre de nylon vendue sous la dénomination "ORGASOL" par la Société ATOCHEM | 15 g |
| - Stéarate de Zinc | 3 g |
| - Pigment d'oxyde de titane et de silice vendu sous la dénomination FLONAC R "TS60C" par la Société KEMIRA | 10 g |
| - Vaseline | 0,7 g |
| - Huile de vaseline | 3,8 g |
| - Lanoline | 0,39 g |
| - Huile de ricin | 0,76 g |
| - Parahydroxybenzoate de propyle | 0,14 g |
| - Talc q.s.p | 100 g |

Cette composition apporte un effet nacrant irisé et un niveau de couvrance approprié.

### EXEMPLE 8 : Fard à joues

| | |
|---|---|
| - Stéarate de Zinc | 3 g |
| - Oxyde de titane | 2 g |
| - Oxyde de fer | 9 g |
| - Mica | 24 g |
| - Poudre de nylon vendue sous la dénomination ORGASOL par la Société ATOCHEM | 15 g |
| - Pigment d'oxyde de titane et de silice vendu sous la dénomination FLONAC R "TS40C" par la Société KEMIRA | 5 g |
| - Huile de vaseline | 3,26 g |
| - Alcool oléique | 0,6 g |
| - Myristate d'isopropyle | 0,43 g |
| - Parahydroxybenzoate de propyle | 0,12 g |
| - Talc q.s.p. | 100 g |

Par comparaison, on reproduit la même formule sans le pigment FLONAC R TS40C.

La composition suivant l'invention présente un effet coloré irisé et une grande douceur au toucher.

### EXEMPLE 9 : Poudre libre pour le visage

| | |
|---|---|
| - Oxyde de fer | 0,5 g |
| - Stéarate de Zinc | 2 g |
| - Oxyde de Zinc | 2 g |
| - Kaolin | 2 g |
| - Poudre de nylon vendue sous la dénomination "ORGASOL" par la Société ATOCHEM | 30 g |
| - Huile de vaseline | 4 g |
| - Pigment d'oxyde de titane et de silice vendu sous la dénomination FLONAC R "TS40C" par la Société KEMIRA | 3 g |
| - Talc q.s.p. | 100 g |

### EXEMPLE 10 : Poudre compactée pour le visage

| | |
|---|---|
| - Oxyde de fer | 6,57 g |
| - Stéarate de Zinc | 4 g |
| - Dioxyde de titane | 2 g |
| - Oxychlorure de bismuth | 10 g |
| - Poudre de nylon vendue sous la dénomination "ORGASOL" par la Société ATOCHEM | 20 g |
| - Huile de vaseline | 3,26 g |
| - Alcool oléique | 0,6 g |
| - Myristate d'isopropyle | 0,43 g |
| - Parahydroxybenzoate de propyle | 0,12 g |
| - Pigment d'oxyde de titane et de silice vendu sous la dénomination FLONAC R "TS40C" par la Société KEMIRA | 5 g |
| - Talc q.s.p. | 100 g |

## Revendications

1. Composition cosmétique pour le maquillage contenant, dans un support cosmétique approprié, un pigment transparent constitué d'au moins 60 % en poids d'oxyde de titane sous forme lamellaire et d'au plus 10 % en poids d'oxyde de silicium, la dimension moyenne des pigments étant comprise entre 1 et 300 µm et l'épaisseur entre 0,001 et 0,3 µm.

2. Composition cosmétique selon la revendication 1, caractérisée par le fait que le pigment transparent est constitué d'au moins 90 % en poids d'oxyde de titane et d'au plus 6 % en poids d'oxyde de silicium.

3. Composition cosmétique selon la revendication 1 ou 2, caractérisée par le fait que le pigment transparent est constitué d'environ 94 % en poids d'oxyde de titane et de 4 % en poids d'oxyde de silicium.

4. Composition selon l'une quelconque des revendications précédentes, caractérisée par le fait que la dimension moyenne du pigment transparent est comprise entre 5 et 50 µm et l'épaisseur entre entre 0,01 et 0,2 µm.

5. Composition cosmétique selon l'une quelconque des revendications précédentes, caractérisée par le fait que le pigment transparent est présent à une concentration comprise entre 0,5 et 30 % et de préférence entre 0,5 et 10 % en poids par rapport au poids total de la composition.

6. Composition cosmétique selon l'une quelconque des revendications 1 à 5, caractérisée par le fait qu'elle contient en outre au moins une charge additionnelle présente à une concentration comprise entre 0,01 et 90 % en poids par rapport au poids total de la composition.

7. Composition cosmétique selon l'une quelconque des revendications précédentes, caractérisée par le fait qu'elle se présente sous forme d'une émulsion huile-dans-eau ou eau-dans-huile, d'une suspension, ou sous forme de poudre libre, de poudre compactée, de solide ou de pâte anhydre ou encore sous forme d'un vernis à ongles.

8. Composition cosmétique selon la revendication 7, caractérisée par le fait que l' émulsion huile-dans-eau ou eau-dans-huile contient un agent tensio-actif en une proportion comprise entre 0,01 et 30 % en poids, la phase huileuse de l'émulsion étant comprise entre 0,1 et 50 % en poids par rapport au poids total de la composition.

9. Composition cosmétique selon la revendication 7, caractérisée par le fait qu'elle se présente sous forme solide ou pâteuse anhydre et contient au moins un agent liant présent en une proportion comprise entre 0,01 et 95 % en poids par rapport au poids total de la composition.

10. Composition cosmétique selon la revendication 7, caractérisée par le fait qu'elle se présente sous forme d'un vernis à ongles contenant :
- de 55 à 90 % en poids d'un système solvant,
- de 5 à 20 % en poids d'une matière filmogène,
- de 2 à 10 % en poids d'un agent plastifiant, et
- de 0,5 à 15 % en poids d'une résine.

11. Composition selon la revendication 10, caractérisée par le fait qu'elle contient de préférence de 0,01 à 10 % en poids du pigment transparent.

## Claims

1. Cosmetic make-up composition containing, in a suitable cosmetic carrier, a transparent pigment consisting of at least 60 % by weight of titanium oxide in lamellar form and at most 10 % by weight of silicon oxide, the mean size of the pigments being between 1 and 300 µm and the thickness between 0.001 to 0.3 µm.

2. Cosmetic composition according to Claim 1, characterized by the fact that the transparent pigment consists of at least 90 % by weight of titanium oxide and at most 6 % by weight of silicon oxide.

3. Cosmetic composition according to Claim 1 or 2, characterized by the fact that the transparent pigment consists of about 94 % by weight of titanium oxide and 4 % by weight of silicon oxide.

4. Composition according to any one of the preceding claims, characterized by the fact that the mean size of the transparent pigment is between 5 and 50 µm and the thickness between between 0.01 and 0.2 µm.

5. Cosmetic composition according to any one of the preceding claims, characterized by the fact that the transparent pigment is present at a concentration of between 0.5 and 30 % and preferably between 0.5 and 10 % by weight relative to the total weight of the composition.

6. Cosmetic composition according to any one of Claims 1 to 5, characterized by the fact that it contains, in addition, at least one additional filler present at a concentration of between 0.01 and 90 % by weight relative to the total weight of the composition.

7. Cosmetic composition according to any one of the preceding claims, characterized by the fact that it is provided in the form of an oil-in-water or water-in-oil emulsion, a suspension or in the form of a loose powder, a compacted powder, a solid or an anhydrous paste or alternatively in the form of a nail varnish.

8. Cosmetic composition according to Claim 7, characterized by the fact that the oil-in-water or water-in-oil emulsion contains a surface-active agent in a proportion of between 0.01 and 30 % by weight, the oily phase of the emulsion being between 0.1 and 50 % by weight relative to the total weight of the composition.

9. Cosmetic composition according to Claim 7, characterized by the fact that it is provided in anhydrous solid or pasty form and contains at least one binding agent present in a proportion of between 0.01 and 95 % by weight relative to the total weight of the composition.

10. Cosmetic composition according to Claim 7, characterized by the fact that it is provided in the form of a nail varnish containing:
- from 55 to 90 % by weight of a solvent system,
- from 5 to 20 % by weight of a film-forming material,
- from 2 to 10 % by weight of a plasticizing agent, and
- from 0.5 to 15 % by weight of a resin.

11. Composition according to Claim 10, characterized by the fact that it preferably contains from 0.01 to 10 % by weight of the transparent pigment.

## Patentansprüche

1. Kosmetische Zubereitung zum Schminken mit einem Gehalt an transparentem Pigment in einem geeigneten kosmetischen Träger, wobei sich das Pigment aus mindestens 60 Gew.-% Titanoxid in Lamellenform und höchstens 10 Gew.-% Siliziumoxid besteht und die durchschnittlichen Abmessungen der Pigmente im Bereich zwischen 1 und 300 µm bei einer Dicke zwischen 0,001 und 0,3 µm liegen.

2. Kosmetische Zubereitung nach Anspruch 1, dadurch gekennzeichnet, daß das transparente Pigment aus mindestens 90 Gew.-% Titanoxid und höchstens 6 Gew.-% Siliziumoxid besteht.

3. Kosmetische Zubereitung nach Anspruch 1 oder 2, dadurch gekennzeichnet, daß das transparente Pigment aus etwa 94 Gew.-% Titanoxid und 4 Gew.-% Siliziumoxid besteht.

4. Zubereitung nach einem der vorstehenden Ansprüche, dadurch gekennzeichnet, daß die durchschnittlichen Abmessungen des transparenten Pigmentes zwischen 5 und 50 µm bei einer Dicke zwischen 0,01 und 0,2 µm betragen.

5. Kosmetische Zubereitungen nach einem der vorstehenden Ansprüche, dadurch gekennzeichnet, daß das transparente Pigment in einer Konzentration zwischen 0,5 und 30 Gew.-%, vorzugsweise zwischen 0,5 und 10 Gew.-% in bezug auf das Gesamtgewicht der Zubereitung vorhanden ist.

6. Kosmetische Zubereitung nach einem der Ansprüche 1 bis 5, dadurch gekennzeichnet, daß sie zusätzlich noch mindestens einen weiteren Träger enthält, der in einer Konzentration zwischen 0,01 und 90 Gew.-% in bezug auf das Gesamtgewicht der Zubereitung vorhanden ist.

7. Kosmetische Zubereitung nach einem der vorstehenden Ansprüche, dadurch gekennzeichnet, daß sie in Form einer Öl-in-Wasser- oder Wasser-in-Öl-Emulsion, einer Suspension oder in Form eines frei fließenden Pulvers oder eines Kompaktpuders in fester Form oder in Form einer wasserfreien Paste oder auch in Form eines Nagellackes vorliegt.

8. Kosmetische Zubereitung nach Anspruch 7, dadurch gekennzeichnet, daß die Öl-in-Wasser- oder Wasser-in-Öl-Emulsion ein Tensid in einem Mengenverhältnis zwischen 0,01 und 30 Gew.-% enthält, wobei die ölige Phase der Emulsion in einer Menge zwischen 0,1 und 50 Gew.-% in bezug auf das Gesamtgewicht der Zubereitung vorliegen.

9. Kosmetische Zubereitungen nach Anspruch 7, dadurch gekennzeichnet, daß sie in fester oder pastenartiger wasserfreier Form vorliegt und mindestens ein Bindemittel enthält, das in einem Mengenverhältnis zwischen 0,01 und 95 Gew.-% in bezug auf das Gesamtgewicht der Zubereitung vorhanden ist.

10. Kosmetische Zubereitung nach Anspruch 7, dadurch gekennzeichnet, daß sie in Form eines Nagellackes vorliegt, der die folgenden Bestandteile enthält:
- 55 bis 90 Gew.-% eines Lösungsmittelsystems,
- 5 bis 20 Gew.-% eines Filmbildners,
- 2 bis 10 Gew.-% eines Weichmachers und
- 0,5 bis 15 Gew.-% eines Kunstharzes.

11. Zubereitung nach Anspruch 10, dadurch gekennzeichnet, daß sie vorzugsweise 0,01 bis 10 Gew.-% transparentes Pigment enthält.
